# EUROPÄISCHE PATENTSCHRIFT

(11) **EP 1 704 892 B1**
(45) Veröffentlichungstag und Bekanntmachung des Hinweises auf die Patenterteilung: **25.08.2010**
(21) Anmeldenummer: 06005472.3
(22) Anmeldetag: 17.03.2006
(51) Int. Cl.: A61N 1/05

(54) **Vorrichtung zur Elektrostimulation**
Electrostimulation device
Dispositif d'électrostimulation

(30) Priorität: 21.03.2005 DE 202005004732 U
(43) Veröffentlichungstag der Anmeldung: 27.09.2006
(73) Patentinhaber: Biobedded Systems GmbH, 46045 Oberhausen (DE)
(72) Erfinder: Hilburg, Andreas, 46045 Oberhausen (DE)
(74) Vertreter: Tongbhoyai, Martin

(56) Entgegenhaltungen:
- EP-A- 0 733 381
- EP-A1- 0 178 514
- FR-A1- 2 767 481
- US-A- 4 881 526
- US-A- 5 370 671

## Beschreibung

Die Erfindung betrifft eine Vorrichtung zur Elektrostimulation und/oder Elektro-Myographie (EMG), die mindestens eine Elektrode aufweist. Die Elektrode ist dabei an einem Elektrodenhalter der Vorrichtung angeordnet.

Es existieren zahlreiche Vorrichtungen zur Elektrostimulation oder Elektro-Myographie , die insbesondere bei der Schmerztherapie und bei der Behandlung gegen Inkontinenz eingesetzt werden. Diese Vorrichtungen weisen jeweils mindestens eine Elektrode auf, die an einem Elektrodenhalter der jeweiligen Vorrichtung angeordnet ist. Die Form der Vorrichtungen bzw. Elektroden ist dabei den anatomischen Gegebenheiten des Anwenders bzw. der Anwenderin angepaßt. Sie werden allerdings häufig für einen einzelnen Anwender bzw. für eine einzelne Anwenderin individuell hergestellt. Sie weisen demnach den Nachteil auf, daß sie für weitere Anwender bzw. Anwenderinnen nicht mehr verwendet werden können. Zudem ist auch eine nachträgliche Anpassung der Form der Vorrichtung bzw. derer Elektrode(n) nicht mehr möglich. Dies ist aber wünschenswert, da auf diese Weise insbesondere die Schmerztherapie und die Inkontinenzbehandlung verbessert werden kann.

Die bekannten Vorrichtungen bzw. deren Elektroden müssen nach jeder Anwendung gereinigt und desinfiziert werden. Jedoch weisen die bekannten Vorrichtungen häufig derart Formen und Aufbauten auf, daß eine Reinigung und Desinfektion nur mit hohem Aufwand möglich ist. Sehr oft ist eine Reinigung und Desinfektion auch nur unzureichend möglich. Hersteller derartiger Vorrichtungen empfehlen zudem, daß eine bestimmte Vorrichtung aus hygienischen Gründen stets nur von ein und derselben Person verwendet werden soll.

Aus der FR-A-2 767 481 ist eine Vorrichtung zur Elektrostimulation mit einer Elektrode bekannt, die an einem Elektrodenhalter angeordnet ist. Die bekannte Vorrichtung weist eine Aufnahme zur Anordnung eines Kontaktelements auf.

Der Erfindung liegt daher die Aufgabe zu Grunde, eine Vorrichtung zur Elektrostimulation und/oder EMG mit mindestens einer Elektrode anzugeben, die leicht zu reinigen und desinfizieren ist sowie deren Form individuell wählbar ist. Ferner ist es wünschenswert, daß die Vorrichtung vom mehreren Personen verwendet werden kann.

Diese Aufgabe wird erfindungsgemäß mit einer Vorrichtung mit den Merkmalen des Anspruchs 1 gelöst. Weitere Ausgestaltungen der Erfindung ergeben sich aus den Merkmalen der Unteransprüche sowie den weiteren in der Beschreibung genannten und in den Figuren gezeigten Merkmale.

Die erfindungsgemäße Vorrichtung zur Elektrostimulation und/oder EMG weist mindestens eine Elektrode auf, die an einem Elektrodenhalter angeordnet ist. Der Elektrodenhalter weist eine erste Elektrodenhalter-Ausdehnung auf. In anderen Worten ausgedrückt besitzt der Elektrodenhalter eine vorgegebene Dimension in einer bestimmten Richtung. Ferner ist an dem Elektrodenhalter mindestens eine Aufnahme vorgesehen, in der ein Kontaktelement anordbar ist. Die Aufnahme weist eine erste Aufnahme-Ausdehnung auf, die mindestens 40% der ersten Elektrodenhalter-Ausdehnung beträgt. Somit entspricht die Aufnahme-Ausdehnung mindestens zwei fünftel der ersten Elektrodenhalter-Ausdehnung. Die Aufnahme ist schlitzförmig ausgebildet und verläuft parallel zur Längsachse des Elektrodenhalters, wobei in der Aufnahme ein Kontaktelement anordbar ist. Die Elektrode ist ferner auf den Elektrodenhalter schiebbar, wobei beim Schieben die Elektrode das Kontaktelement berührt.

Die erfindungsgemäße Vorrichtung weist den Vorteil auf, daß die Elektrode bzw. die Elektroden austauschbar an dem Elektrodenhalter angeordnet sind. Da über einen großen Bereich des Elektrodenhalters ein elektrischer Kontakt zwischen der Elektrode bzw. den Elektroden und einem Kontaktelement gewährleistet ist, ist es möglich, die Elektrode bzw. die Elektroden an einer Vielzahl von Orten an dem Elektrodenhalter anzuordnen. Jeder dieser Orte muß nur so gewählt sein, daß ein elektrischer Kontakt zwischen Elektrode und Kontaktelement herstellbar ist. Von Vorteil ist auch, daß bei der Erfindung die Form der Elektrode bzw. der Vorrichtung je nach Anwendung frei wählbar ist. Somit ist praktisch die Form der Elektrode bzw. Vorrichtung individuell an die anatomischen Gegebenheiten des Anwenders bzw. der Anwenderin anpaßbar. Je nach Einsatzgebiet können insbesondere längliche oder ringförmige Elektroden verwendet werden.

Die Erfindung weist zudem den Vorteil auf, daß sie sich leicht in ihre einzelnen Bauteile, insbesondere den Elektrodenhalter und die Elektrode(n), zerlegen läßt, die dann unkompliziert und mit geringem Aufwand (beispielsweise in einem handelsüblichen Sterilisator) gereinigt werden können.

Bei einer Ausführungsform der Erfindung ist vorgesehen, daß die erste Aufnahme-Ausdehnung mindestens 50% der ersten Elektrodenhalter-Ausdehnung beträgt. Vorzugsweise beträgt die Aufnahme-Ausdehnung mindestens 60%, bei einer weiteren Ausführungsform mindestens 70% der ersten Elektrodenhalter-Ausdehnung. Mit anderen Worten ausgedrückt, erstreckt sich die Aufnahme für ein Kontaktelement vorzugsweise mindestens über die Hälfte der Ausdehnung des Elektrodenhalters.

Es hat sich gezeigt, daß eine erste Elektrodenhalter-Ausdehnung im Bereich zwischen 50mm und 70mm besonders bevorzugt ist. Desweiteren ist vorzugsweise vorgesehen, daß die erste Elektrodenhalter-Ausdehnung und die erste Aufnahme-Ausdehnung sich in eine erste Richtung erstrecken. Somit sind die Elektrodenhalter-Ausdehnung und die erste Aufnahme-Ausdehnung in dieselbe Richtung orientiert.

Bei einer weiteren Ausführungsform der Erfindung ist es vorgesehen, in der Aufnahme ein Kontaktelement anzuordnen, das eine erste Kontaktelement-Ausdehnung in die erste Richtung aufweist. Diese erste Kontaktelement-Ausdehnung ist etwas geringer als die erste Aufnahme-Ausdehnung, vorzugsweise um ca. 1 % bis 10% geringer als die erste Aufnahme-Ausdehnung. Somit weist das Kontaktelement eine Ausdehnung derart auf, daß es sicher in der Aufnahme des Elektrodenhalters angeordnet werden kann.

Das Kontaktelement weist vorzugsweise ein erstes Ende und ein zweites Ende auf. An dem ersten Ende des Kontaktelements sind an einer ersten Seite ein Eingriffsmittel und an einer zweiten Seite eine Ausnehmung angeordnet. Die Ausnehmung ist in der Aufnahme an einem am Elektrodenhalter angeordneten Sicherungsmittel gehalten und gewährleistet, daß das Kontaktelement sich nicht entlang der ersten Kontaktelement-Ausdehnung in der Aufnahme verschiebt. Dies ist deshalb von Vorteil, da an dem ersten Ende vorzugsweise eine Steckverbindung angeordnet ist. An dieser Steckverbindung ist ein Stecker anbringbar, der das Kontaktelement mit einer Versorgungseinrichtung (Spannungsversorgung/Stromversorgung/Meßeinrichtung) verbindet. Beim Anbringen des Steckers an die Steckverbindung wird das Kontaktelement somit nicht verrutschen. Die Funktion des Eingriffmittels wird weiter unten erläutert.

Das Kontaktelement weist vorzugsweise auch eine zweite Kontaktelement-Ausdehnung auf, die sich in einer zweiten Richtung senkrecht zur ersten Richtung der ersten Kontaktelement-Ausdehnung erstreckt und die zwischen 1% und 5% der ersten Kontaktelement-Ausdehnung beträgt. Die zweite Kontaktelement-Ausdehnung ist die Dicke des Kontaktelements. Im Vergleich zur ersten Kontaktelement-Ausdehnung, die der Länge des Kontaktelements entspricht, ist sie sehr gering.

Bei einer weiteren Ausführungsform der Erfindung weist das Kontaktelement eine dritte Kontaktelement-Ausdehnung in einer dritten Richtung auf, die senkrecht zur ersten Richtung und senkrecht zur zweiten Richtung orientiert ist. Die dritte Kontaktelement-Ausdehnung entspricht der Höhe des Kontaktelements. Vorzugsweise verjüngt sich die dritte Kontaktelement-Ausdehnung in einem Bereich in Richtung zum zweiten Endes hin, wobei sich dieser Bereich von dem zweiten Ende bis zu einem Punkt erstreckt, der zu dem ersten Ende einen Abstand von mindestens ein Drittel, bevorzugt aber mindestens der Hälfte der ersten Kontaktelement-Ausdehnung aufweist. Diese Verjüngung des Kontaktelements ist derart in der Aufnahme angeordnet, daß sie mit einem Boden der Aufnahme als Wippe wirkt. Durch diese Wippe ist es möglich, das bereits oben genannte Eingriffsmittel, das an dem ersten Ende angeordnet ist, mit einem Isolator und/oder einem Handgriff in Eingriff zu bringen, so daß dieser an dem Elektrodenhalter fixiert ist.

Ferner ist es vorzugsweise vorgesehen, an dem zweiten Ende des Kontaktelements ein Federelement anzuordnen. Durch Bewegen des Federelements in Richtung des Elektrodenhalters wird das Eingriffsmittel in Eingriff mit dem Isolator und/oder Handgriff gebracht.

Bei einer weiteren Ausführungsform der Erfindung ist der Elektrodenhalter zylinderförmig bzw. stabförmig ausgebildet. Vorzugsweise ist vorgesehen, daß der Elektrodenhalter ein erstes Elektrodenhalter-Ende und ein zweites Elektrodenhalter-Ende aufweist. An dem ersten Elektrodenhalter-Ende ist ein Gewindeabschnitt und an dem zweiten Elektrodenhalter-Ende ist eine Verbindungseinrichtung zur Herstellung eines elektrischen Kontakts angeordnet. An dem Gewindeabschnitt ist vorzugsweise ein erstes Abschlußstück angeordnet. Hingegen ist an dem zweiten Elektrodenhalter-Ende ein zweites Abschlußstück vorgesehen, in dem die bereits oben genannte Steckverbindung angeordnet ist. Das erste Abschlußstück ist vorzugsweise konisch ausgebildet und dient als Einführmittel der erfindungsgemäßen Vorrichtung in Körperöffnungen. Das zweite Abschlußstück ist insbesondere als Griffstück ausgebildet, das mit dem Eingriffsmittel, wie bereits oben erläutert, in Eingriff ist. Zwischen dem ersten und dem zweiten Abschlußstück werden die zu verwendenden Elektroden und mögliche zu verwendende Isolatoren sicher gehalten. Bei einer Ausführungsform ist vorgesehen, daß zwei durch mindestens einen Isolator getrennte Elektroden an dem Elektrodenhalter angeordnet sind.

Vorzugsweise weist die erfindungsgemäße Vorrichtung mindestens zwei Aufnahmen zur Anordnung von Kontaktelementen an dem Elektrodenhalter auf. Bevorzugt ist eine Ausführungsform der Erfindung, bei der drei oder mehr Aufnahmen zur Anordnung von Kontaktelementen an dem Elektrodenhalter unter einem Winkel von <=120° angeordnet sind.

Wie oben erwähnt, ist die Form der Elektroden und Isolatoren, die an dem Elektrodenhalter angeordnet werden, frei wählbar. Bei vielen Anwendungen sind ringförmige Elektroden vorgesehen. Die Erfindung ist allerdings nicht auf diese Form der Elektroden beschränkt. Vielmehr können die Elektroden jegliche Form aufweisen, die für die entsprechende Anwendung und Anatomie des Anwenders bzw. der Anwenderin notwendig ist. Hierzu zählen auch längliche Elektroden, die voneinander durch längliche Isolatoren getrennt sind. Diese Anordnung der Elektroden und Isolatoren wird ebenfalls durch die oben beschriebene Klemmung zwischen den beiden Abschlußstücken gehalten.

Nachfolgend wird die Erfindung anhand von Ausführungsbeispielen mittels Figuren näher beschrieben.

Es zeigen
- Fig. 1: eine schematische Darstellung einer Vorrichtung zur Elektrosti- mulation und/oder EMG gemäß der Erfindung;
- Fig. 2: einen Elektrodenhalter der Vorrichtung gemäß Fig. 1;
- Fig. 3: ein Kontaktelement der Vorrichtung gemäß Fig. 1;
- Fig. 4: ein Griffstück der Vorrichtung gemäß Fig. 1;
- Fig. 5: ein Abschlußstück der Vorrichtung gemäß Fig. 1;
- Fig. 6: einen ringförmigen Isolator der Vorrichtung gemäß Fig. 1;
- Fig. 7: eine ringförmige Elektrode der Vorrichtung gemäß Fig. 1; und
- Fig. 8: eine weitere Vorrichtung zur Elektrostimulation gemäß der Erfindung.

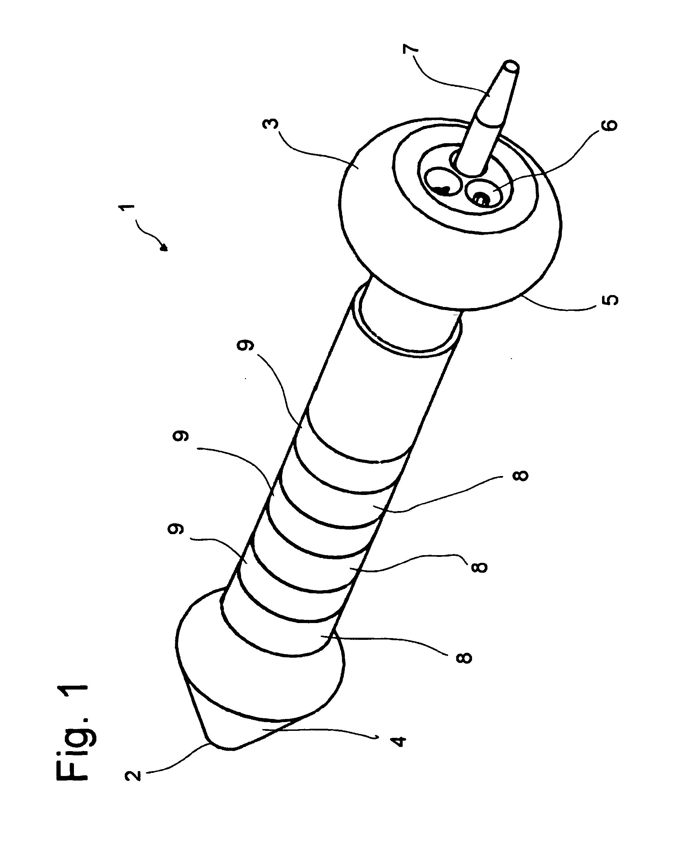

Fig. 1 zeigt eine schematische Darstellung eines ersten Ausführungsbeispiels der erfindungsgemäßen Vorrichtung 1 zur Elektrostimulation. Die Vorrichtung 1 ist eine Vorrichtung zur Inkontinenzbehandlung. Die Erfindung ist allerdings nicht auf diese Anwendung eingeschränkt. Vielmehr ist die erfindungsgemäße Vorrichtung 1 zu jedem Zweck einsetzbar, bei der eine Elektrostimulation oder eine EMG benötigt wird.

Die Vorrichtung 1 weist ein erstes Ende 2 und ein zweites Ende 3 auf. Das erste Ende 2 umfaßt ein konisch gebildetes erstes Abschlußstück 4. Das erste Abschlußstück 4 bestimmt die Einführrichtung der Vorrichtung 1 in entsprechende Körperöffnungen des Anwenders bzw. der Anwenderin. Das zweite Ende 3 der Vorrichtung 1 weist ein zweites Abschlußstück 5 auf, an dem eine Verbindungseinrichtung 6 angeordnet ist. Die Verbindungseinrichtung 6 dient zur Aufnahme von Steckern 7, die die Vorrichtung 1 über Kabel mit einer Versorgungs- und Steuereinheit sowie einer Meßeinheit (für EMG) (nicht dargestellt) verbindet.

Die Vorrichtung 1 weist mehrere Elektroden 8 auf, die bei dem dargestellten Ausführungsbeispiel der erfindungsgemäßen Vorrichtung ringförmig ausgebildet sind. Die Elektroden 8 sind voneinander durch ebenfalls ringförmig ausgebildete Isolatoren 9 getrennt. Es wird ausdrücklich darauf hingewiesen, daß die Erfindung nicht auf ringförmige Elektroden eingeschränkt ist. Vielmehr ist die Form der Elektroden und der Isolatoren frei wählbar, wie weiter unten nochmals erläutert wird.

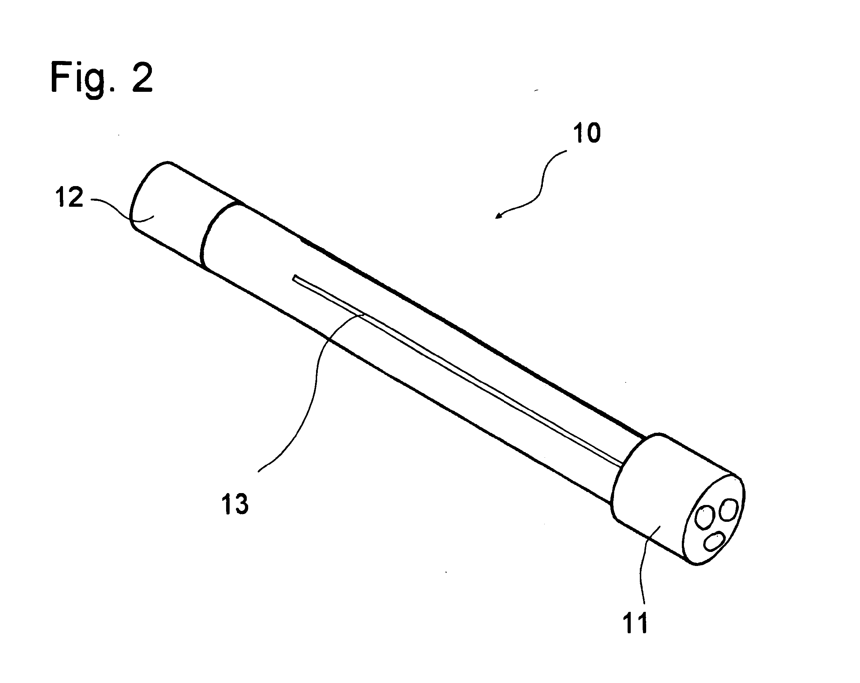

Fig. 2 zeigt eine schematische Darstellung eines stabförmigen Elektrodenhalters 10 der Vorrichtung 1, an dem das erste Abschlußstück 4, das zweite Abschlußstück 5, die Elektroden 8 sowie die Isolatoren 9 angeordnet sind (in Fig. 2 nicht dargestellt). Der Elektrodenhalter 10 weist ein erstes Ende 11 und ein zweites Ende 12 auf. Das erste Ende 11 weist die bereits obengenannte Verbindungseinrichtung 6 für die Stecker 7 auf. Hingegen weist das zweite Ende 12 einen Gewindeabschnitt auf. An diesem Gewindeabschnitt wird das erste Abschlußstück 4 angeordnet, das ebenfalls ein Gewinde aufweist. Somit wird das erste Abschlußstück 4 durch eine Schraubverbindung mit dem Elektrodenhalter 10 verbunden.

Der Elektrodenhalter 10 des hier dargestellten Ausführungsbeispiels ist ca. 102mm lang. Über seinen Umfang sind unter 120° drei Aufnahmen 13 angeordnet, die schlitzförmig ausgebildet sind und deren Längsausdehnung parallel zur Längsachse des Elektrodenhalters 10 verläuft. Die Längsausdehnung jeder Aufnahme 13 beträgt ca. 63mm.

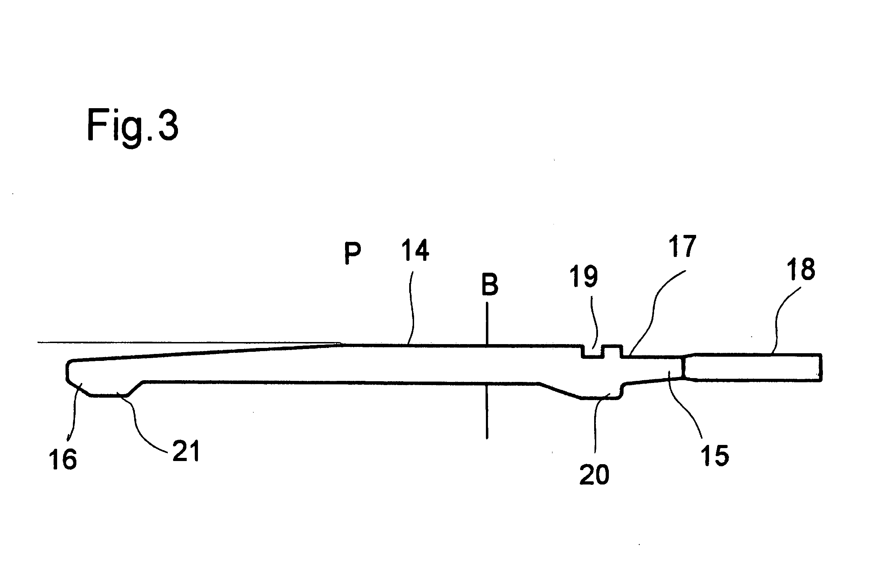

In jeder der Aufnahmen 13 ist jeweils ein Kontaktelement 14 angeordnet, das in Fig. 3 näher dargestellt ist. Das Kontaktelement 14 weist eine Längsausdehnung auf, die etwas geringer als die Längsausdehnung der Aufnahme 13 ausgebildet ist. Bei dem dargestellten Ausführungsbeispiel beträgt die Längsausdehnung des Kontaktelements 14 ca. 50mm. Das Kontaktelement 14 weist ein erstes Ende 15 und ein zweites Ende 16 auf. Das erste Ende 15 ist mit einem Steg 17 versehen, auf dem eine Steckerbuchse 18 angeordnet ist. Diese Steckerbuchse 18 ist Teil der Verbindungseinrichtung 6. In die Steckerbuchse 18 wird einer der Stecker 7 angeordnet, um das Kontaktelement 14 und somit auch die Elektroden 8, wie später noch erläutert wird, mit Spannung/Strom zu versorgen oder einen Meßstrom (EMG) weiterzuleiten.

Das erste Ende 15 des Kontaktelements 14 ist ferner auf einer Seite mit einer Ausnehmung 19 versehen. Die andere Seite des Endes 15 ist mit einem Eingriffsmittel 20 versehen. Die Funktionen der Ausnehmung 19 und des Eingriffmittels 20 werden weiter unten noch näher beschrieben.

Das Kontaktelement 14 dieses Ausführungsbeispiels ist recht dünn ausgebildet. Die Dicke des Kontaktelements 14 beträgt ca. 0,5 bis 2mm. Im Bereich des Endes 15 weist das Kontaktelement 14 eine Breite B von ca. 4 bis 5mm auf. Die Breite B verjüngt sich ab einem Punkt P in Richtung zum zweiten Ende 16 des Kontaktelements 14 hin. Auf der Seite, an der auch das Eingriffsmittel 20 angeordnet ist, ist an dem zweiten Ende 16 ein Federmittel 21 angeordnet.

Wie oben erwähnt, ist das Kontaktelement 14 in der Aufnahme 13 des Elektrodenhalters 10 angeordnet. Dabei wird das Kontaktelement 14 derart in der Aufnahme 13 eingesetzt, daß die Ausnehmung 19 in Richtung der Längsachse des Elektrodenhalters 10 zeigt. Die Ausnehmung 19 greift dabei in einen Elektrodenhalter-Steg (nicht dargestellt) ein. Im Anschluß daran wird die Steckerbuchse 18 an den Steg 17 des Kontaktelements 14 angeordnet. Durch den Eingriff der Ausnehmung 19 in den Elektrodenhalter-Steg wird gewährleistet, daß bei Anordnen des Steckers 7 in der Steckerbuchse 18 das Kontaktelement 14 nicht innerhalb der Aufnahme 13 verrutscht.

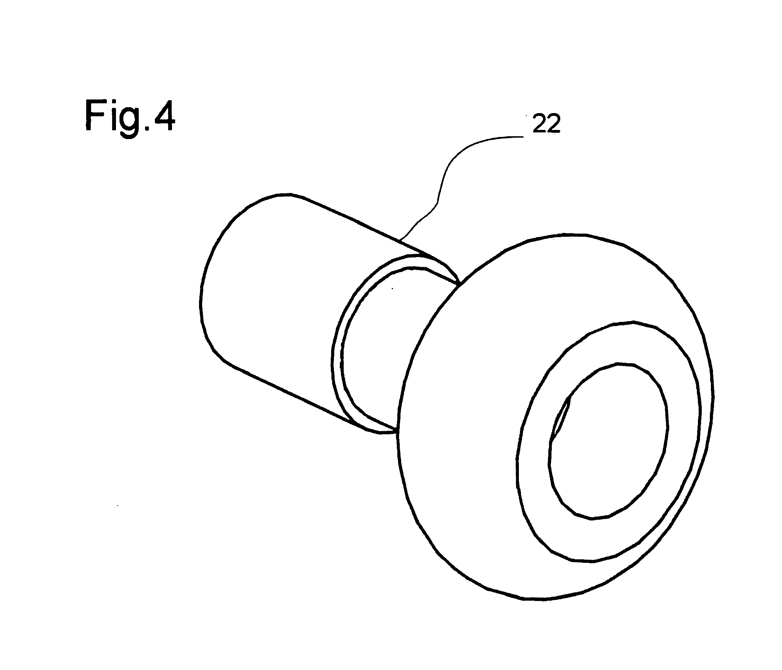

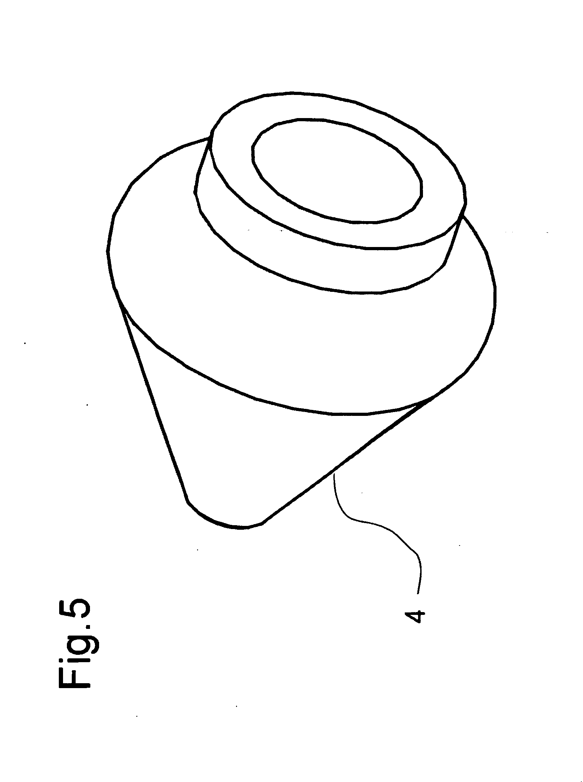

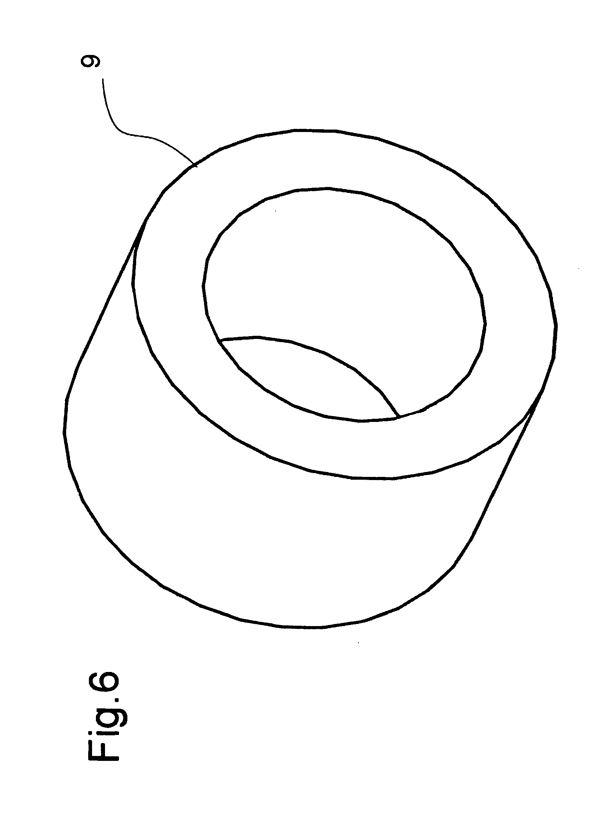

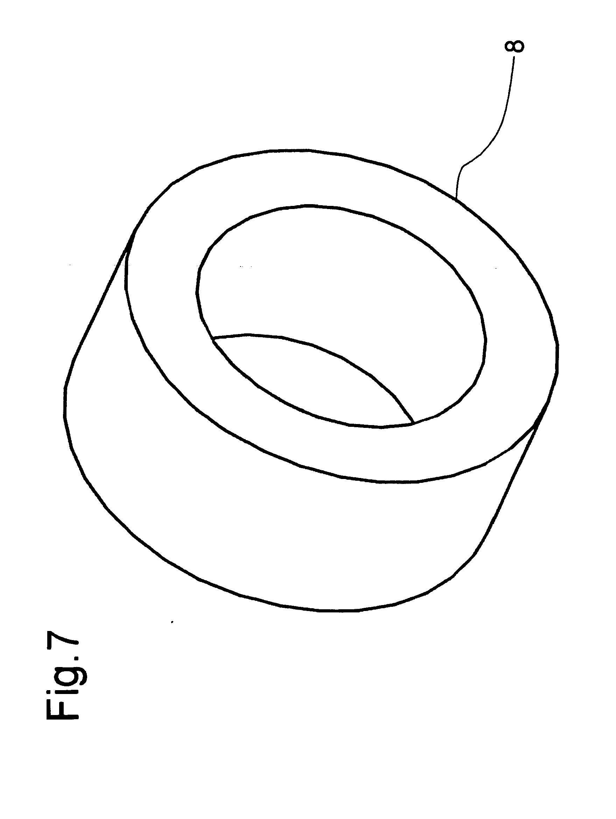

Nachfolgend wird nun der Zusammenbau der Vorrichtung 1 näher beschrieben. Dabei wird davon ausgegangen, daß in jeder Aufnahme 13 ein Kontaktelement 14 sowie die entsprechende Steckerbuchse 18 bereits montiert ist. Zunächst wird ein Griffstück 22 (vgl. Fig. 4) auf das zweite Ende 12 des Elektrodenhalters 10 und dann anschließend auf das erste Ende 11 geschoben. Alternativ hierzu kann das Griffstück 22 aber auch direkt auf das erste Ende 11 des Elektrodenhalters 10 geschoben werden. Anschließend werden die Elektroden 8 und Isolatoren 9 (vgl. Fig. 6 und 7) über den Gewindeabschnitt 12 auf den Elektrodenhalter 10 in der gewünschten Reihenfolge geschoben. Beim Schieben berühren die Elektroden 8 und Isolatoren 9 das Federelement 21 des Kontaktelements 14. Aufgrund der oben beschriebenen verjüngten Ausbildung kommt es dabei zu einer Wippbewegung, so daß das Federelement 21 und somit das Kontaktelement 14 in Richtung der Längsachse des Elektrodenhalters 10 gedrückt wird. Die Anordnung der Elektroden 8 und Isolatoren 9 ist somit problemlos möglich. Mindestens ein Bereich des Kontaktelements 14 ist dann stets in Kontakt mit mindestens einer Elektrode 8. Zudem wird durch die Bewegung des Kontaktelements 14 aufgrund der verjüngten Ausbildung das Eingriffsmittel 20 von dem Elektrodenhalter 10 derart weg bewegt, daß das Eingriffsmittel 20 mit dem Griffstück 22 in Eingriff kommt. Das Griffstück 22 ist somit fixiert und kann sich weder in Längsrichtung bewegen noch um die Längsachse des Elektrodenhalters 10 drehen. Sobald sämtliche Elektroden 8 und Isolatoren 9 an dem Elektrodenhalter 10 angeordnet sind, wird das erste Abschlußstück 4 (Fig. 5) auf das zweite Ende 12 des Elektrodenhalters 10 mit dem Gewindeabschnitt aufgeschraubt. Dabei kann die Vorrichtung 1 an dem Griffstück 22 gehalten werden, das gegenüber dem Elektrodenhalter 10 fixiert ist.

Die erfindungsgemäße Vorrichtung 1 ist nach deren Gebrauch wieder in ihre Einzelteile zerlegbar. Hierzu wird durch Halten der Vorrichtung 1 an dem Griffstück 22 das erste Abschlußstück 4 von dem Elektrodenhalter 10 geschraubt und anschließend sämtliche Elektroden 8 und Isolatoren 9 von dem Elektrodenhalter 10 wieder entfernt. Im Anschluß daran wird auch das Griffstück 22 von dem Elektrodenhalter 10 entfernt. Alle Teile sind nun sehr einfach zu reinigen. Sie lassen sich insbesondere in handelsüblichen Sterilisatoren reinigen.

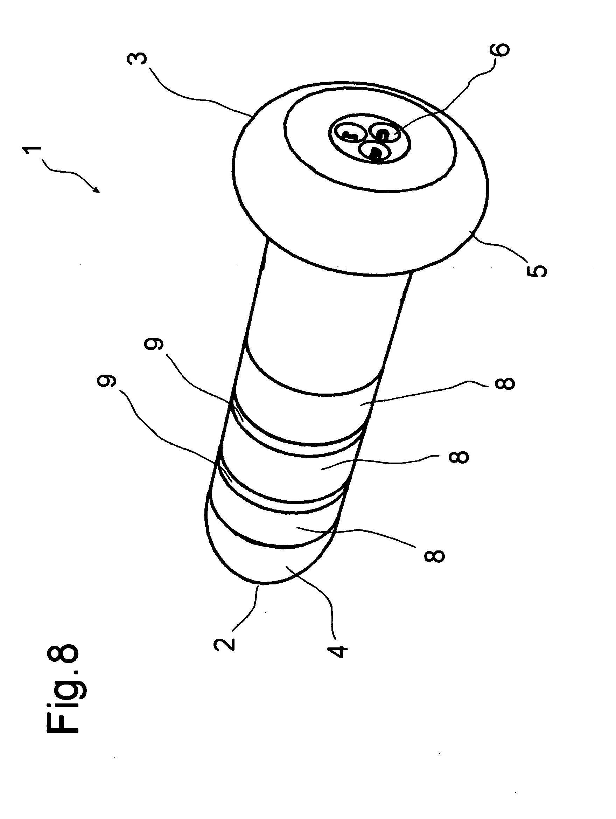

Die Erfindung weist zudem den Vorteil auf, daß man die Form und Größe der Elektroden 8 und Isolatoren 9 individuell wählen kann. Je nach Anwendungsgebiet und anatomischen Gegebenheiten können die Elektroden 8 und Isolatoren 9 gewählt und an dem Elektrodenhalter 10 angeordnet werden. Die Befestigung und sichere Anordnung erfolgt wiederum durch das erste Abschlußstück 4 und das Griffstück 22. Fig. 8 zeigt beispielsweise eine gegenüber der Ausführungsform gemäß Fig. 1 im Durchmesser größere Vorrichtung 1 (zweites Ausführungsbeispiel). In anderen Worten ausgedrückt, ermöglicht es die Erfindung, auf einen Elektrodenhalter 10 unterschiedliche Elektroden 8 und Isolatoren 9 anzuordnen. Die Erfindung ermöglicht daher nicht nur Variationsmöglichkeiten hinsichtlich der Form, sondern auch hohe Einsparungen beispielsweise im Klinikbetrieb, da der Elektrodenhalter 10 mit den entsprechenden Elektroden mehrfach und nach Reinigung bei unterschiedlichen Personen zur Anwendung kommen kann.

### Bezugszeichenliste

- 1: Vorrichtung
- 2: erstes Ende der Vorrichtung
- 3: zweites Ende der Vorrichtung
- 4: erstes Abschlußstück
- 5: zweites Abschlußstück
- 6: Verbindungseinrichtung
- 7: Stecker
- 8: Elektrode
- 9: Isolator
- 10: Elektrodenhalter
- 11: erstes Ende Elektrodenhalter
- 12: zweites Ende Elektrodenhalter
- 13: Aufnahme
- 14: Kontaktelement
- 15: erstes Ende des Kontaktelements
- 16: zweites Ende des Kontaktelements
- 17: Steg
- 18: Steckerbuchse
- 19: Ausnehmung
- 20: Eingriffsmittel
- 21: Federelement
- 22: Griffstück

## Patentansprüche

1. Vorrichtung (1) zur Elektrostimulation und/oder Elektro-Myographie, mit mindestens einer Elektrode (8), die an einem Elektrodenhalter (10) angeordnet ist, wobei
- der Elektrodenhalter (10) eine erste Elektrodenhalter-Ausdehnung aufweist,
- an dem Elektrodenhalter (10) mindestens eine Aufnahme (13) zur Anordnung eines Kontaktelements (14) angeordnet ist, und wobei die Aufnahme (13) eine erste Aufnahme-Ausdehnung aufweist, die mindestens 40% der ersten Elektrodenhalter-Ausdehnung beträgt,
**dadurch gekennzeichnet,**
- **dass** die Aufnahme (13) schlitzförmig ausgebildet ist und parallel zur Längsachse des Elektrodenhalters verläuft, wobei in der Aufnahme ein Kontaktelement anordbar ist, und dass
- die Elektrode (8) auf den Elektrodenhalter (10) schiebbar ist, wobei beim Schieben die Elektrode das Kontaktelement berührt.

2. Vorrichtung nach Anspruch 1, **dadurch gekennzeichnet, daß** die erste Aufnahme-Ausdehnung mindestens 50% der ersten Elektrodenhalter-Ausdehnung beträgt.

3. Vorrichtung nach Anspruch 1 oder 2, **dadurch gekennzeichnet, daß** die erste Aufnahme-Ausdehnung mindestens 60% der ersten Elektrodenhalter-Ausdehnung beträgt.

4. Vorrichtung nach mindestens einem der vorangehenden Ansprüche,
**dadurch gekennzeichnet, daß** die erste Aufnahme-Ausdehnung mindestens 70% der ersten Elektrodenhalter-Ausdehnung beträgt.

5. Vorrichtung nach mindestens einem der vorangehenden Ansprüche,
**dadurch gekennzeichnet, daß** die erste Elektrodenhalter-Ausdehnung im Bereich zwischen 50mm und 70mm liegt.

6. Vorrichtung nach mindestens einem der vorangehenden Ansprüche,
**dadurch gekennzeichnet, daß** die erste Elektrodenhalter-Ausdehnung und die erste Aufnahme-Ausdehnung sich in eine erste Richtung erstrecken.

7. Vorrichtung nach mindestens einem der vorangehenden Ansprüche,
**dadurch gekennzeichnet, daß** in der Aufnahme (13) ein Kontaktelement (14) angeordnet ist, das eine erste Kontaktelement-Ausdehnung in der ersten Richtung aufweist, wobei die erste Kontaktelement-Ausdehnung um 1 % bis 10% der ersten Aufnahme-Ausdehnung geringer als die erste Aufnahme-Ausdehnung ist.

8. Vorrichtung nach Anspruch 7, **dadurch gekennzeichnet, daß** das Kontaktelement (14) ein erstes Ende (15) und ein zweites Ende (16) aufweist, wobei an dem ersten Ende (15) an einer ersten Seite ein Eingriffsmittel (20) und an einer zweiten Seite eine Ausnehmung (19) angeordnet sind.

9. Vorrichtung nach Anspruch 8, **dadurch gekennzeichnet, daß** an dem ersten Ende (15) eine Steckverbindung (17, 18) angeordnet ist.

10. Vorrichtung nach einem der Ansprüche 7 bis 9, **dadurch gekennzeichnet, daß** das Kontaktelement (14) eine zweite Kontaktelement-Ausdehnung aufweist, die sich in einer zweiten Richtung senkrecht zur ersten Richtung der ersten Kontaktelement-Ausdehnung erstreckt und die zwischen 1% und 5% der ersten Kontaktelement-Ausdehnung beträgt.

11. Vorrichtung nach einem der Ansprüche 7 bis 10, **dadurch gekennzeichnet, daß** das Kontaktelement (14) eine dritte Kontaktelement-Ausdehnung (B) in einer dritten Richtung, die senkrecht zur ersten Richtung und senkrecht zur zweiten Richtung orientiert ist, aufweist, wobei die dritte Kontaktelement-Ausdehnung sich in einem Bereich in Richtung des zweiten Endes (16) des Kontaktelements (14) hin verjüngt und wobei der Bereich sich von dem zweiten Ende (16) bis zu einem Punkt (P) erstreckt, der zu dem ersten Ende (15) einen Abstand von mehr als die Hälfte der ersten Kontaktelement-Ausdehnung aufweist.

12. Vorrichtung nach einem der Ansprüche 7 bis 11, **dadurch gekennzeichnet, daß** das zweite Ende (16) ein Federelement (21) aufweist.

13. Vorrichtung nach mindestens einem der vorangehenden Ansprüche,
**dadurch gekennzeichnet, daß** der Elektrodenhalter (10) stabförmig ausgebildet ist.

14. Vorrichtung nach mindestens einem der vorangehenden Ansprüche,
**dadurch gekennzeichnet, daß** der Elektrodenhalter (10) ein erstes Elektrodenhalter-Ende (12) und ein zweites Elektrodenhalter-Ende (11) aufweist, wobei an dem ersten Elektrodenhalter-Ende (12) ein Gewindeabschnitt und an dem zweiten Elektrodenhalter-Ende (11) eine Verbindungseinrichtung (6) zur Herstellung eines elektrischen Kontakts angeordnet sind.

15. Vorrichtung nach mindestens einem der vorangehenden Ansprüche,
**dadurch gekennzeichnet, daß** mindestens zwei Aufnahmen (13) zur Anordnung von Kontaktelementen (14) an dem Elektrodenhalter (10) angeordnet sind.

16. Vorrichtung nach Anspruch 15, **dadurch gekennzeichnet, daß** drei Aufnahmen (13) zur Anordnung von Kontaktelementen (14) an dem Elektrodenhalter (10) unter einem Winkel von 120° angeordnet sind.

17. Vorrichtung nach mindestens einem der vorangehenden Ansprüche,
**dadurch gekennzeichnet, daß** zwei durch mindestens einen Isolator (9) getrennte Elektroden (8) auf dem Elektrodenhalter (10) angeordnet sind.

18. Vorrichtung nach mindestens einem der vorangehenden Ansprüche,
**dadurch gekennzeichnet, daß** an dem ersten Elektrodenhalter-Ende (12) ein erstes Abschlußstück (4) und an dem zweiten Elektrodenhalter-Ende (11) ein zweites Abschlußstück (5) angeordnet sind.

## Claims

1. A device (1) for electrostimulation and/or electromyography, having at least one electrode (8) arranged on an electrode holder (10),
- the electrode holder (10) comprising a first electrode holder dimension,
- at least one receptacle (13) being arranged on the electrode holder (10) for arrangement of a contact element (14), and the receptacle (13) comprising a first receptacle dimension which amounts to at least 40% of the first electrode holder dimension,
**characterised in that**
- the receptacle (13) is of slot-shaped construction and extends parallel to the longitudinal axis of the electrode holder, wherein a contact element may be arranged in the receptacle, and **in that**
- the electrode (8) may be pushed onto the electrode holder (10), wherein the electrode contacts the contact element on pushing.

2. A device according to claim 1, **characterised in that** the first receptacle dimension amounts to at least 50% of the first electrode holder dimension.

3. A device according to claim 1 or claim 2, **characterised in that** the first receptacle dimension amounts to at least 60% of the first electrode holder dimension.

4. A device according to at least one of the preceding claims, **characterised in that** the first receptacle dimension amounts to at least 70% of the first electrode holder dimension.

5. A device according to at least one of the preceding claims, **characterised in that** the first electrode holder dimension lies in the range between 50 mm and 70 mm.

6. A device according to at least one of the preceding claims, **characterised in that** the first electrode holder dimension and the first receptacle dimension extend in a first direction.

7. A device according to at least one of the preceding claims, **characterised in that** a contact element(14) is arranged in the receptacle (13), which contact element comprises a first contact element dimension in the first direction, the first contact element dimension being smaller than the first receptacle dimension by 1% to 10% of the first receptacle dimension.

8. A device according to claim 7, **characterised in that** the contact element (14) comprises a first end (15) and a second end (16), wherein at the first end (15) an engagement means (20) is arranged on a first side and a recess (19) is arranged on a second side.

9. A device according to claim 8, **characterised in that** a plug and socket connection (17, 18) is arranged at the first end (15).

10. A device according to any one of claims 7 to 9, **characterised in that** the contact element (14) comprises a second contact element dimension, which extends in a second direction perpendicular to the first direction of the first contact element dimension and which amounts to between 1% and 5% of the first contact element dimension.

11. A device according to any one of claims 7 to 10, **characterised in that** the contact element (14) comprises a third contact element dimension (B) in a third direction, which is oriented perpendicular to the first direction and perpendicular to the second direction, the third contact element dimension tapering in one region towards the second end (16) of the contact element (14) and the region extending from the second end (16) up to a point (P) which is at a distance from the first end (15) of more than half the first contact element dimension.

12. A device according to any one of claims 7 to 11, **characterised in that** the second end (16) comprises a spring element (21).

13. A device according to any one of the preceding claims, **characterised in that** the electrode holder (10) is of rod-shaped construction.

14. A device according to at least one of the preceding claims, **characterised in that** the electrode holder (10) comprises a first electrode holder end (12) and a second electrode holder end (11), a threaded portion being arranged at the first electrode holder end (12) and a connecting means (6) for producing an electrical contact being arranged at the second electrode holder end (11).

15. A device according to at least one of the preceding claims, **characterised in that** at least two receptacles (13) for the arrangement of contact elements (14) are arranged on the electrode holder (10).

16. A device according to claim 15, **characterised in that** three receptacles (13) for the arrangement of contact elements (14) are arranged on the electrode holder (10) at an angle of 120°.

17. A device according to at least one of the preceding claims, **characterised in that** two electrodes (8) separated by at least one insulator (9) are arranged on the electrode holder (10).

18. A device according to at least one of the preceding claims, **characterised in that** a first end piece (4) is arranged at the first electrode holder end (12) and a second end piece (5) is arranged at the second electrode holder end (11).

## Revendications

1. Dispositif (1) pour l'électrostimulation et/ou l'électromyographie, comprenant au moins une électrode (8), qui est disposée sur un porte-électrode (10), dans lequel
- le porte-électrode (10) présente une première extension du porte-électrode,
- au moins un logement (13) pour l'agencement d'un élément de contact (14) est disposé sur le porte-électrode (10) et le logement (13) présente une première extension de logement qui représente au moins 40 % de la première extension du porte-électrode,
**caractérisé en ce que**
- le logement (13) est conçu en forme de fente et s'étend parallèlement à l'axe longitudinal du porte-électrode, un élément de contact pouvant être disposé dans le logement, et **en ce que**
- l'électrode (8) peut être glissé sur le porte-électrode (10), l'électrode touchant l'élément de contact lors du glissement.

2. Dispositif selon la revendication 1, **caractérisé en ce que** la première extension de logement représente au moins 50 % de la première extension du porte-électrode.

3. Dispositif selon la revendication 1 ou 2, **caractérisé en ce que** la première extension de logement représente au moins 60 % de la première extension du porte-électrode.

4. Dispositif selon au moins l'une quelconque des revendications précédentes, **caractérisé en ce que** la première extension de logement représente au moins 70 % de la première extension du porte-électrode.

5. Dispositif selon au moins l'une quelconque des revendications précédentes, **caractérisé en ce que** la première extension du porte-électrode se situe dans la plage comprise entre 50 mm et 70 mm.

6. Dispositif selon au moins l'une quelconque des revendications précédentes, **caractérisé en ce que** la première extension du porte-électrode et la première extension du logement s'étendent dans une première direction.

7. Dispositif selon au moins l'une quelconque des revendications précédentes, **caractérisé en ce que** dans le logement (13) est disposé un élément de contact (14) qui présente une première extension de l'élément de contact dans la première direction, la première extension de l'élément de contact étant inférieure de 1 % à 10 % de la première extension de logement par rapport à la première extension de logement.

8. Dispositif selon la revendication 7, **caractérisé en ce que** l'élément de contact (14) présente une première extrémité (15) et une seconde extrémité (16), un moyen d'engagement (20) et un évidement (19) étant disposés sur la première extrémité (15) respectivement sur un premier côté et sur un second côté.

9. Dispositif selon la revendication 8, **caractérisé en ce qu'**une liaison à enfichage (17, 18) est disposée sur la première extrémité (15).

10. Dispositif selon l'une quelconque des revendications 7 à 9, **caractérisé en ce que** l'élément de contact (14) présente une seconde extension de l'élément de contact qui s'étend dans une seconde direction perpendiculairement à la première direction de la première extension de l'élément de contact et qui représente entre 1 % et 5 % de la première extension de l'élément de contact.

11. Dispositif selon l'une quelconque des revendications 7 à 10, **caractérisé en ce que** l'élément de contact (14) présente une troisième extension d'élément de contact (B) dans une troisième direction, qui est orientée perpendiculairement à la première direction et perpendiculairement à la seconde direction, la troisième extension de l'élément de contact se rétrécissant dans une zone en direction de la seconde extrémité (16) de l'élément de contact (14) et la zone s'étendant de la seconde extrémité (16) jusqu'à un Point (P), qui présente par rapport à la première extrémité (15) une distance supérieure à la moitié de la première extension de l'élément de contact.

12. Dispositif selon l'une quelconque des revendications 7 à 11, **caractérisé en ce que** la seconde extrémité (16) présente un élément de ressort (21).

13. Dispositif selon au moins l'une quelconque des revendications précédentes, **caractérisé en ce que** le porte-électrode (10) est conçu en forme de barre.

14. Dispositif selon au moins l'une quelconque des revendications précédentes, **caractérisé en ce que** le porte-électrode (10) présente une première extrémité de porte-électrode (12) et une seconde extrémité de porte-électrode (11), une partie filetée étant disposée sur la première extrémité de porte-électrode (12) et un dispositif de liaison (6) pour l'établissement d'un contact électrique sur la seconde extrémité de porte-électrode (11).

15. Dispositif selon au moins l'une quelconque des revendications précédentes, **caractérisé en ce qu'**au moins deux logements (13) pour l'agencement d'éléments de contact (14) sont disposés sur le porte-électrode (10).

16. Dispositif selon la revendication 15, **caractérisé en ce que** trois logements (13) pour l'agencement d'éléments de contact (14) sont disposés sur le porte-électrode (10) sous un angle de 120°.

17. Dispositif selon au moins l'une quelconque des revendications précédentes, **caractérisé en ce que** deux électrodes (8) séparées par au moins un isolateur (9) sont disposées sur le porte-électrode (10).

18. Dispositif selon au moins l'une quelconque des revendications précédentes, **caractérisé en ce qu'**une première pièce de fermeture (4) et une seconde pièce de fermeture (5) sont disposées respectivement sur la première extrémité du porte-électrode (12) et sur la seconde extrémité du porte-électrode (11).
